Europäisches Patentamt

European Patent Office

Office européen des brevets

(19)

(11) **EP 1 201 231 A2**

(12) **EUROPEAN PATENT APPLICATION**

(43) Date of publication:
**02.05.2002 Bulletin 2002/18**

(51) Int Cl.⁷: **A61K 9/16**

(21) Application number: **01309255.6**

(22) Date of filing: **31.10.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.10.2000 JP 2000333932**

(71) Applicants:
- **National Institute of Advanced Industrial Science and Technology Tokyo 100-8921 (JP)**
- **Ito, Atsuo Tsukuba-shi, Ibaraki 305-8562 (JP)**

(72) Inventors:
- **Ito, Atsuo, c/o Nat. Inst. of Advanced Ind. Sci. Tsukuba-shi, Ibaraki 305-8562 (JP)**

- **Otsuka, Makoto Kobe-shi, Hyogo 658-0001 (JP)**
- **Ichinose, Noboru Yokohama-shi, Kanagawa 236-0033 (JP)**
- **Sakurai, Tokoha Tsukuba-shi, Ibaraki 305-0031 (JP)**
- **Fukasawa, Koushirou Nakano-ku, Tokyo 164-0014 (JP)**
- **Kamo, Michimasa Shiki-shi, Saitama 353-0006 (JP)**

(74) Representative: **Maschio, Antonio D Young & Co, 21 New Fetter Lane London EC4A 1DA (GB)**

(54) **Suspension or particle-solvent mixture system of zinc-containing calcium phosphate microparticle, and therapeutic agent for treating zinc deficiency**

(57) A suspension or a particle-solvent mixture system, which contains microparticles of zinc-containing calcium phosphate with particular chemical composition, specifically, microparticles of sparingly soluble zinc-containing calcium phosphate, and a therapeutic agent for treating zinc deficiency are provided. Particularly, a suspension or particle-solvent mixture system is prepared by mixing microparticles of zinc-containing calcium phosphate consisting of from 0.6 ppm to 13% by weight of zinc, 33-57% by weight of $P_2O_5$, 10-65% by weight of CaO and 0-28% by weight of $H_2O$ with a water-miscible or water-immiscible solvent. Such a suspension or particle-solvent mixture system can be used as a therapeutic agent for treating zinc deficiency.

**EP 1 201 231 A2**

**Description**

Field of the Invention

The present invention relates to a suspension or particle-solvent mixture system which contains zinc-containing calcium phosphate with particular chemical composition, specifically, sparingly soluble zinc-containing calcium phosphate. The present invention also relates to a therapeutic agent for treating diseases or deficiency state caused by zinc deficiency or marginal zinc deficiency such as bone disease, dermatophathia, dysgeusia or immunopathy. The therapeutic agent contains, as an active ingredient, sparingly soluble zinc-containing calcium phosphate which provides sustained release of zinc, and is useful for treatment of diseases or deficiency states caused by zinc deficiency or marginal zinc deficiency.

Background of the Invention

Zinc is known to stimulate osteoblast as well as suppress osteoclast activity *in vivo* (see, Res. Exp. Med. 186, 337, 1986; Bio. Pharmacol., 36, 4007, 1987; Biochem. Pharmacol., 48, 1225, 1994; J. Biomed. Mater. Res. 50, 184, 2000). These activities indicate the ability of zinc to promote ossification.

It is also known that shortage of zinc leads to many diseases or deficiency states, including promoted bone resorption, reduced osteogenesis, dermatophathia, dysgeusia or immunopathy (see, Endocrinol. 114, 1860, 1984; Newer trace elements in nutrition, p.255, 1971; Am. J. Clin. Nutr., 68, 447S, 1998).

For health maintenance, it is said that an adult requires about 15mg of zinc per day. A lifestyle that involves an unbalanced diet or a high frequency of consuming processed foodstuffs makes it difficult for an adult to take the amount of zinc to be required. Accordingly, zinc tends to be one of the metal elements in which adults are deficient. Recently, it has been reported that the population of patients with dermatopathia or dysgeusia is increasing.

Examples of methods for compensating for a deficiency in zinc include those using zinc chloride, zinc sulfate, zinc phosphate, zinc hydrogenphosphate (PCT/US91/05496), zinc oxide (08.12.88 AU 1849/88), zinc acexamate (Japanese Patent Application Laid-Open No. Hei 10-218767), L- carnosine zinc salt (Japanese Patent Application Laid-Open No. Hei 3-120257), alanyl-L-histidinato zinc (12.04.91GB910783), amino acid chelates (PCT/US87/00310), zinc citrate, zinc glycinate (12.04.91GB9107833) or water soluble zinc compound (Japanese Patent Application Laid-Open No. Sho 61-282317). In these methods, it is proposed that the above-described compound is administered orally or parenterally as a drug.

Administration of these conventionally known compounds for treatment, however, causes sudden elevation of zinc level within the tissue in the vicinity of the site to which the compound is administered (e.g., in buccal cavity, gastrointestinal tract or injection site) or in the affected tissue, followed by gradual decrease in the zinc level with time. Such a dramatic change in zinc level is repeated every time the compound is administered. Too high zinc level in the tissue in the vicinity of the site of administration (e.g., in buccal cavity, gastrointestinal tract or injection site) or in the affected tissue, i.e., overdose of zinc, sometimes causes a side-effect (toxicity), resulting in an undesirable effect.

Accordingly, we developed, after intense study to overcome the disadvantages in the prior art, a novel zinc-containing calcium phosphate ceramics for slowly releasing zinc which contained a reduced amount of zinc and provided long-term sustained release of a constant amount of zinc, thereby preventing overdose of zinc (see, Japanese Patent No. 3,143,660; U. S. Patent No. 6,090,732). This ceramics enables sustained release of zinc, thereby preventing uptake of an excess amount of zinc. Additionally, this ceramics have high biocompatibility and thus can be used as a surgical implant material for hard tissue. Accordingly, this ceramics was able to solve the above-mentioned problems.

This zinc-containing calcium phosphate ceramics which provides sustained release of zinc can continuously release a non-excess amount of zinc slowly over a long period of time in the site in need of zinc as long as the ceramics is present in a body. Moreover, since this ceramics has biocompatibility, it has enabled appropriate treatment of zinc-related diseases such as zinc deficiency. However, a process for manufacturing such a zinc-containing calcium phosphate ceramics which provides sustained release of zinc involves shape-forming and sintering steps. Therefore, the process is very complicated, and when such a ceramics is used, it is required to make operative invasions of the body of the patient, and to fix the ceramics to a particular part of the body.

Summary of the Invention

An object of the invention is to provide a therapeutic agent which enables treatment of zinc deficiency without requiring operative invasions by preparing a suspension or a particle-solvent mixture system that contains microparticles of sparingly soluble zinc-containing calcium phosphate which provides sustained release of zinc, and then formulating it into any dosage form including those for oral administration or injection, plasters, suppositories or ointments.

We succeeded in preparing a suspension or particle-solvent mixture system by preparing a sparingly soluble zinc-containing calcium phosphate compound with a predetermined low level of zinc, granulating the compound, and dispersing and suspending the granules into a liquid. By administering the suspension or particle-solvent mixture system to an animal such as rat, continuous sustained release of zinc can be provided in its body without administering an excess amount of zinc, thereby supplementing the body with zinc. We also found that such a suspension has high biocompatibility after administration.

Based on these findings, we invented a therapeutic agent for treating zinc deficiency in the present invention by suspending microparticles of sparingly soluble zinc-containing calcium phosphate in a solvent to prepare a suspension or particle-solvent mixture system. The therapeutic agent has the following advantages:

- it can be administered to supplement a body with zinc;
- particularly, it can slowly release zinc over a long period of time to supplement the body with zinc without administering an excess amount of zinc; and
- it has biocompatibility with cells and tissues when used *in vivo*.

The present invention provides:

(1) a suspension or particle-solvent mixture system which comprises a liquid containing a microparticle of zinc-containing calcium phosphate consisting of from 0.6 ppm to 13% by weight of zinc, 33-57% by weight of $P_2O_5$, 10-65% by weight of CaO and 0-28% by weight of $H_2O$;

(2) a suspension or particle-solvent mixture system which comprises a liquid containing a microparticle of zinc-containing calcium phosphate consisting of from 120 ppm to 13% by weight of zinc, 33-57% by weight of $P_2O_5$, 10-65% by weight of CaO and 0-28% by weight of $H_2O$;

(3) the suspension or particle-solvent mixture system according to (1) or (2) above wherein said microparticle of zinc-containing calcium phosphate is one or more compounds selected from the group consisting of zinc-containing hydroxyapatite, zinc-containing poorly-crystallized apatite, zinc-containing $\alpha$-tricalcium phosphate, zinc-containing $\beta$-tricalcium phosphate, zinc-containing calcium hydrogenphosphate, and $CaZn_2(PO_4)_2 \cdot nH_2O$ ($0 \leq n \leq 2$);

(4) the suspension or particle-solvent mixture system according to any one of (1)-(3) above, wherein at least one osteogenic compound selected from the group consisting of vitamin D, $\alpha$-calcidol, estrogen-related preparations, calcitonin, bisphosphonate and calcium containing preparations is further contained;

(5) the suspension or particle-solvent mixture system according to any one of (1)-(4) above, wherein said liquid is a water-miscible solvent selected from the group consisting of physiological saline solution, an aqueous solution of 2.5% by weight or less of sodium chloride, Ringer's solution, purified water, distilled water for injection, distilled water, physiological salt solution, propylene glycol, ethanol, and a mixture of propylene glycol or ethanol with one or more of these solutions or waters;

(6) the suspension or particle-solvent mixture system according to any one of (1)-(4) above, wherein said liquid is selected from the group consisting of polyethylene glycol (Macrogol) and water-immiscible solvent including triglyceride, safflower oil, soybean oil, sesame oil, rape seed oil and peanut oil;

(7) the suspension or particle-solvent mixture system according to any one of (1)-(6) above wherein said suspension or particle-solvent mixture system is used for a therapeutic agent for treating zinc deficiency; and

(8) the suspension or particle-solvent mixture system according to (4) above, wherein said suspension or particle-solvent mixture system is used for a therapeutic agent for treating zinc deficiency and osteogenetic agent.

This specification includes part or all of the contents as disclosed in the specification and/or drawings of Japanese Patent Application No. 2000-333932, which is a priority document of the present application.

Brief Description of the Drawings

Figure 1 shows the effects of intramuscular injection of a suspension of zinc-containing $\beta$-tricalcium phosphate microp articles containing 6.17% and 12.05% by weight of zinc on the bone density of rats with zinc-deficiency-type osteoporosis.

Figure 2 shows the effects of intramuscular injection of a suspension of zinc-containing $\beta$-tricalcium phosphate microparticles containing 6.17% and 12.05% by weight of zinc on the bone strength of rats with zinc-deficiency-type osteoporosis.

Figure 3 is a photographic view showing the tissue from rats with zinc-deficiency-type osteoporosis one week after a suspension of zinc-containing $\beta$-tricalcium phosphate microparticles containing 12.05% by weight of zinc was intramuscularly injected.

Figure 4 is a photographic view showing the tissue from rats with zinc-deficiency-type osteoporosis one week after a suspension of zinc-free β -tricalcium phosphate microparticles was intramuscularly injected.

Detailed Description of the Invention

The suspension or the particle-solvent mixture system according to the present invention contains microparticles of zinc-containing calcium phosphate with particular chemical composition, specifically, microparticles of sparingly soluble zinc-containing calcium phosphate compounds.

As used herein, the term "suspension" means a liquid containing particles that sediment at a sedimentation velocity of 0.1 cm/s or smaller. At this range of sedimentation velocity, it takes 80 seconds or more for the particles in the liquid to move downward (sink) a distance of 8 cm vertically through the liquid.

As used herein, the term "particle-solvent mixture system" means a liquid in which all particles sediment at a sedimentation velocity larger than 0.1 cm/s. At this range of sedimentation velocity, it takes less than 80 seconds for the particles in the liquid to move downward (sink) a distance of 8 cm vertically through the liquid.

As used herein, the term "sparingly soluble calcium phosphate" means calcium phosphate that has solubility of less than 0.05 mol per liter (calcium concentration) in water at pH7 at 25°C. Calcium phosphate is sparingly soluble except for monocalcium phosphate and monocalcium phosphate monohydrate. The sparingly soluble calcium phosphate includes calcium hydrogenphosphate, octacalcium phosphate, amorphous calcium phosphate, tricalcium phosphate, whitlockite, hydroxyapatite, calcium deficient hydroxyapatite, tetracalcium phosphate and the mixtures thereof. Tricalcium phosphate includes $\alpha$-tricalcium and β -tricalcium phosphate.

As used herein, the term "calcium phosphate" means calcium phosphate including sparingly soluble calcium phosphate as described above and soluble calcium phosphate with solubility of 0.05 mol per liter or more (calcium concentration) in water at pH7 at 25°C.

As used herein, the term "calcium hydrogenphosphate" means calcium hydrogenphosphate dihydrate, calcium hydrogenphosphate hemihydrate and calcium hydrogenphosphate anhydrate.

As used herein, the term "poorly-crystallized hydroxyapatite" refers to hydroxyapatite that is precipitated from a liquid at a temperature lower than 100°C and that is unheated or heated at 700°C or lower after the precipitation. The liquid from which the poorly-crystallized hydroxyapatite precipitates includes aqueous solution, nonaqueous solvent and the mixtures thereof.

The sparingly soluble zinc-containing calcium phosphate compound can be synthesized by using phosphate component, calcium component, zinc component, and optionally $H_2O$ component.

Particularly, it can be obtained by subjecting calcium compound or ion, phosphate compound or phosphate ion, and zinc compound or ion to solid or liquid phase reaction or to mechanochemical reaction.

For solid phase reaction, phosphate source, calcium source and zinc source may be allowed to react at a high temperature. Particular examples of phosphate source includes ammonium phosphate, calcium phosphate, phosphoric acid anhydride, calcium hydrogenphosphate, glycerophosphoric acid and calcium glycerophosphate. Examples of calcium source includes calcium carbonate, calcium hydroxide, calcium phosphate, calcium oxide, calcium nitrate, calcium glycerophosphate, calcium lactate, calcium acetate and calcium ethoxide. Examples of zinc source includes zinc oxide, zinc nitrate, zinc phosphate, zinc carbonate, zinc hydroxide, zinc lactate and zinc acetate. Zinc-containing calcium phosphate itself may also be used as a source.

For mechanochemical reaction, phosphate source, calcium source and zinc source may be subjected to mechanochemical reaction by grinding or crushing, and optionally heated. Phosphate source, calcium source and zinc source which can be used may include those described above for solid phase reaction. Zinc-containing calcium phosphate itself may also be used.

For liquid phase reaction, any phosphate source may be used which, when dissolved in water, can release phosphate ion in an acidic or neutral aqueous solution. Particular examples of such phosphate source includes phosphoric acid anhydride, phosphoric acid and phosphate. Phosphoric acid may be used after purification with organic solvent such as alcohol since it may generally contain impurities. Phosphates include potassium phosphate, ammonium phosphate, calcium dihydrogenphosphate, glycerophosphoric acid, calcium glycerophosphate and calcium phosphate.

Any calcium source may be used which, when dissolved in water, can release calcium ions in a neutral or acidic aqueous solution. Particular examples of such calcium source includes calcium hydroxide, calcium carbonate, calcium phosphate, calcium nitrate, calcium chloride, calcium glycerophosphate, calcium lactate and calcium acetate.

Any zinc source may be used which, when dissolved in water, can release zinc ions in a neutral or acidic aqueous solution. Particular examples of such zinc source may include zinc nitrate, zinc hydroxide, zinc oxide, zinc carbonate, zinc acetate and zinc lactate. Zinc-containing calcium phosphate itself may also be used as a zinc source.

Target products can also be obtained by adding zinc compound to a solution which precipitates calcium phosphates such as hydroxyapatite.

For preparing sparingly soluble zinc-containing calcium phosphate by liquid phase reaction, sol-gel process using alcohol may be used in place of the above-described aqueous solution reaction process. For example, calcium ethoxide, phosphoric acid and zinc acetate are allowed to react in alcohol under nitrogen atmosphere.

Examples of sparingly soluble zinc-containing calcium phosphate compound obtained by solid phase or mechanochemical reaction using the above-described phosphate, calcium and zinc sources may include zinc-containing poorly-crystallized apatite, zinc-containing hydroxyapatite, zinc-containing $\alpha$-tricalcium phosphate, zinc-containing $\beta$-tricalcium phosphate or $CaZn_2(PO_4)_2 \cdot nH_2O$ (n=0), and a mixture of at least two of these compounds.

Liquid phase reaction using the above-described phosphate, calcium and zinc sources may give precipitate comprising sparingly soluble zinc-containing calcium phosphate compound. Examples of the thus obtained compound includes zinc-containing hydroxyapatite, zinc-containing poorly-crystallized apatite, zinc-containing $\beta$-tricalcium phosphate, zinc-containing calcium hydrogenphosphate, zinc-containing amorphous calcium phosphate or $CaZn_2(PO_4)_2 \cdot nH_2O$ ($1 \leq n \leq 2$), and a mixture of at least two of these compounds.

Mixing the phosphate, calcium and zinc sources at a (Ca+Zn)/P molar ratio of 0.61 or lower may give precipitate consisting of soluble zinc-containing calcium phosphates such as zinc-containing monocalcium phosphate, zinc-containing monocalcium phosphate monohydrate or the mixtures thereof. In this case, the soluble zinc-containing calcium phosphate can be converted into sparingly soluble zinc-containing calcium phosphate including zinc-containing calcium phosphate glass and zinc-containing calcium metaphosphate by heating the soluble zinc-containing calcium phosphate at 200 °C or higher to obtain zinc-containing calcium metaphosphate, and at 980 °C or higher to obtain zinc-containing calcium phosphate glass.

Sparingly soluble zinc-containing calcium phosphate used in the present invention may contain zinc at an amount of from 0.6ppm to 13% by weight, and preferably from 120ppm to 13% by weight. The amount of each of the source materials used may be selected such that the resulting target product contains a desirable amount of zinc.

Product obtained by solid phase or mechanochemical reaction using the above-described phosphate, calcium and zinc sources may be then granulated. Product obtained by liquid phase reaction may be separated by filtration, dried, optionally heated, and then granulated to form a microparticle.

Temperature for the heating step in the above-described solid phase reaction or after the liquid phase reaction depends on the particle to be formed. Microp articles of zinc-containing amorphous calcium phosphate, zinc-containing poorly-crystallized apatite, zinc-containing calcium hydrogenphosphate, or $CaZn_2(PO_4)_2 \cdot nH_2O$ (n=1 or 2) may be prepared without heating step. In this case, precipitates may be separated by filtration, dried, and then granulated without heating step. This is because heating results in conversion of zinc-containing amorphous calcium phosphate into zinc-containing tricalcium phosphate or zinc-containing poorly-crystallized apatite into zinc-containing hydroxyapatite, or dehydration of zinc-containing calcium hydrogenphosphate to produce calcium pyrophosphate with low biocompatibility or $CaZn_2(PO_4)_2 \cdot nH_2O$ (n= 1 or 2) to produce $CaZn_2(PO_4)_2$.

Heating step for preparing zinc-containing hydroxyapatite may be typically performed at 700-1200°C, and preferably at 700-1000°C in the presence of oxygen, since heating at temperatures below 700°C may result in zinc-containing poorly crystallized hydroxyapatite while heating at temperatures above 1200°C may result in decomposition of zinc-containing hydroxyapatite into zinc-containing $\alpha$-tricalcium phosphate and/or zinc oxide phase.

Heating step for preparing zinc-containing $\beta$-tricalcium phosphate may be typically performed at 700-1250°C, and preferably at 700-1050°C in the presence of oxygen, since heating at temperatures below 700°C may result in production of amorphous calcium phosphate or poorly-crystallized zinc-containing Ca-deficient hydroxyapatite. Upper limit of heating temperature may be determined depending on the phase-transition temperature at which zinc-containing $\beta$-tricalcium phosphate is partially changed to zinc-containing $\alpha$-tricalcium phosphate. The phase-transition temperature depends on the amount of zinc contained, e.g., 1130°C for 0 ppm, 1180°C for 300ppm, or 1250°C for 0.7% by weight of zinc. Heating at 1250°C or higher may be possible for 0.7% by weight or higher of zinc but result in overgrowth of particle size, increase in crystallinity, and reduction in zinc-releasing rate with no particular advantage. Therefore, the heating temperature may preferably be within 700-1250°C.

Heating step for preparing zinc-containing $\alpha$-tricalcium phosphate may be typically performed at 1150-1500°C, and preferably at 1200-1450°C in the presence of oxygen. Heating at temperatures below 1150°C may not provide a single phase of zinc-containing $\alpha$-tricalcium phosphate with zinc content of 0.6ppm or more but rather a single phase of zinc-containing $\beta$-tricalcium phosphate or a mixture of zinc-containing $\beta$-tricalcium phosphate and zinc-containing $\alpha$-tricalcium phosphate. On the other hand, heating at temperatures above 1500°C will not produce any zinc-containing $\alpha$-tricalcium phosphate but merely zinc-containing super $\alpha$-tricalcium phosphate as a stable phase regardless of the zinc content (E.R. Eric & F.A. Hummel, Inorganic Chem., 6, 524, 1967).

Heating step for preparing a microparticle mixture of zinc-containing $\beta$-tricalcium phosphate and zinc-containing $\alpha$-tricalcium phosphate may be typically performed at 1150-1500°C, and preferably at 1150-1400°C in the presence of oxygen. This range was determined based on the range of temperature at which zinc-containing $\beta$-tricalcium phosphate is stably co-present with zinc-containing $\alpha$-tricalcium phosphate, which is from 1130±5°C to 1500°C (E.R. Eric & F.A. Hummel, Inorganic Chem., 6, 524, 1967).

Heating step for preparing a microparticle mixture of zinc-containing $\beta$-tricalcium phosphate and zinc-containing hydroxyapatite may be typically performed at 700-1200°C, and preferably at 800-1200°C in the presence of oxygen, since heating at temperatures below 700°C may provide zinc-containing hydroxyapatite with low crystallinity while heating at temperatures above 1200°C may result in decomposition of zinc-containing hydroxyapatite to produce zinc-containing $\alpha$-tricalcium phosphate and/or zinc oxide phase.

Heating step for preparing $CaZn_2(PO_4)_2 \cdot nH_2O(n=0)$ may be typically performed at 400-1050°C, and preferably at 600-1050°C in the presence of oxygen. This range was determined since heating at 385°C or lower temperatures may result in residual crystallized water of $CaZn_2(PO_4)_2 \cdot nH_2O$ and co-presence of impurities such as $CaZn_2(PO_4)_2 \cdot nH_2O(n=1$ or $2)$ or the like, while heating at temperatures above 1050°C may result in melting of $CaZn_2(PO_4)_2 \cdot nH_2O$ (n = 0) (B. Darko et al., Am. Mineralogist., 66, 843, 1981; E.R. Eric & F.A. Hummel, Inorganic Chem., 6, 524, 1967).

Heating step for preparing a microparticle mixture of $CaZn_2(PO_4)_2 \cdot nH_2O(n= 0)$ and zinc-containing $\beta$-tricalcium phosphate may be typically performed at 700-1050°C, and preferably at 750-950°C, since heating at temperatures below 700°C may result in production of amorphous calcium phosphate or poorly-crystallized zinc-containing Ca-deficient hydroxyapatite, while heating at temperatures above 1050°C may result in melting of $CaZn_2(PO_4)_2 \cdot nH_2O$ (n = 0).

The thus obtained product may be ground in a grinder, sieved and elutriated to obtain microparticles of 0.1 μm-2000 μm (particle size), and preferably of 5 μm-100 μm. Any suitable grinder can be used which can produce such a range of particle size. Typically, sieving may be employed to collect microparticles of 300 μm-32 μm (particle size). Particles smaller than 32 μm may be obtained by elutriating in water-miscible or immiscible solvent.

As described above, sparingly soluble zinc-containing calcium phosphate can be obtained as a single compound or a mixture of compounds. Particularly, it may be obtained as a compound selected from the group consisting of zinc-containing amorphous calcium phosphate, zinc-containing hydroxyapatite, zinc-containing poorly-crystallized apatite, zinc-containing $\alpha$-tricalcium phosphate, zinc-containing $\beta$-tricalcium phosphate, zinc-containing calcium hydrogenphosphate, and $CaZn_2(PO_4)_2 \cdot nH_2O$ ($0 \leq n \leq 2$); a mixture of at least two selected from these compounds; or a compound consisting of a particular one selected from these compounds which can be obtained by adding other compound(s) to the particular compound and converting other compound(s) to the particular one.

According to the present invention, these compounds may be used alone or in combination. Therefore, these compounds can be used separately as a single phase or as a mixture without isolation. Each component can be identified by powder X-ray analysis.

Composition of obtained product can be varied by the process for preparing sparingly soluble zinc-containing calcium phosphate compound according to the present invention using a variety of ratios of the source materials to be used. Further, a certain substance or substances can be added to the compound to prepare microparticles containing a particular substance.

For example, calcium source (calcium hydroxide) may be allowed to react with phosphate source and zinc source (zinc nitrate) in water at a Ca:P:Zn molar ratio of 1.53: 1.20: 0.27-1.26: 1.20:0.54 and heated at 850°C in the air to obtain a mixture of zinc-containing $\beta$-tricalcium phosphate and $CaZn_2(PO_4)_2$ as a powder of sparingly soluble zinc-containing calcium phosphate. The thus obtained mixture may be then added with tricalcium phosphate and sintered for solid phase reaction, resulting in zinc-containing $\beta$-tricalcium phosphate powder.

For example, calcium source (calcium oxide) can be allowed to react with phosphate source and zinc source (zinc nitrate hexahydrate) in water at a mass ratio of (83.36: 97.99: 4.05)-(84.10: 97.99: 0.09) while keeping the molar ratio of (Ca+Zn)/P=1.50, and the resulting precipitate separated by filtration, dried and then heated in the air at 1400°C to obtain zinc-containing an $\alpha$-tricalcium phosphate powder with 0.5% to 120ppm by weight of zinc.

For example, calcium source (calcium hydroxide) can be allowed to react with phosphate source and zinc source (zinc nitrate) in water at a Ca:P:Zn molar ratio of 1.996: 1.20: 0.004 to obtain a suspension of poorly-crystallized zinc-containing hydroxyapatite which contains 0.13% by weight of zinc. The suspension may be then added with hydroxyapatite powder, fully stirred, separated by filtration and heated at 850°C so that solid phase reaction occurs between the hydroxyapatite and the poorly-crystallized zinc-containing hydroxyapatite containing 0.13% by weight of zinc, thereby obtaining zinc-containing hydroxyapatite powder which contains 0.6ppm-less than 0.13% by weight of zinc.

For example, calcium source (calcium hydroxide) can be allowed to react with phosphate source and zinc source (zinc nitrate) in water while keeping the molar ratio of (Ca+Zn)/P=1.67 to obtain precipitate of zinc-containing poorly-crystallized hydroxyapatite up to 2.0% of Zn/Ca molar ratio. In this case, the zinc-containing poorly-crystallized hydroxyapatite may contain 1.3% by weight of zinc. Rapid reaction in water while keeping the molar ratio of (Ca+Zn)/P =1.5 can give precipitate of zinc-containing amorphous calcium phosphate.

For example, calcium source (calcium hydroxide) can be allowed to react with phosphate source and zinc source (zinc nitrate) in water at room temperature at a Ca:P:Zn molar ratio of 1:2:2, and separated by filtration to give $CaZn_2(PO_4)_2 \cdot nH_2O(n=2)$. When the separated product is heated at 100-300°C, then $CaZn_2(PO_4)_2 \cdot nH_2O(n=1$ or $2)$, or a mixture containing $CaZn_2(PO_4)_2 \cdot nH_2O(n=1)$ may be obtained. Alternatively, when the separated product is heated at 400-1050°C, then $CaZn_2(PO_4)_2 \cdot nH_2O(n=0)$ may be obtained.

For example, calcium source (calcium carbonate) may be allowed to react with phosphate source and zinc source (zinc nitrate) in water at room temperature at a Ca:P:Zn molar ratio of 1:1:0.004-0.02 to obtain zinc-containing calcium hydrogenphosphate dihydrate.

The zinc-releasing rate of the compound according to the present invention may depend on the particle size of the compound when it is used as a zinc-releasing drug comprising the compound dispersed in a liquid. Particle size larger than the above-described range may result in the amount of zinc released being smaller. Further, a compound of such a larger particle size may not be able to flow through the bore of a syringe needle or a catheter. As a result, operative invasion of the subject body may be required for parenteral administration. Only particles of the above-described range can provide a desirable releasing rate of a component such as zinc. Use of a smaller particle size than the above-described range may substantially result in colloidal solution, which may undesirably cause rapid release of zinc, adsorption and/or denaturation of biological components such as proteins, and/or sudden change in local pH in body fluid.

The thus obtained product may contain zinc at an amount of from 0.6ppm to 13% by weight, and preferably from 120ppm to 13% by weight. From this range of zinc content, the range of content of ZnO component is calculated by multiplying the zinc content by 1.244 that corresponds to the formula weight ratio of ZnO to Zn. Based on the content of ZnO component calculated from this range of zinc content, the contents of $P_2O_5$, CaO and $H_2O$ components may be selected within the range of 33-57 % by weight of $P_2O_5$, 10-65% by weight of CaO and 0-28% by weight of $H_2O$, so that the sum of the percentages of the ZnO component, $P_2O_5$ component, CaO component and $H_2O$ component equals 100%.

The microparticle may contain zinc at an amount of from 0.6ppm to 13% by weight for the following reasons.

Zinc is present as an essential trace element at an amount of 0.8ppm-1.1ppm by weight in saliva, body fluid and blood obtained from a healthy human, while the corresponding amount is 0.5ppm-0.8ppm by weight in a patient with zinc deficiency or marginal zinc deficiency.

It should be noted that, for zinc-mediated therapeutic and supplemental purposes, the suspension or particle-solvent mixture system according to the present invention should contain microparticles with a zinc concentration higher than that contained in body fluid from a patient with zinc deficiency or marginal zinc deficiency.

Accordingly, the lower limit of zinc content in the microparticle is to be set to 0.6ppm by weight. In other words, none or little therapeutic and/or supplemental effect may be expected on zinc deficiency when less than 0.6ppm by weight of zinc is used.

On the other hand, the above-described upper limit of zinc content in the microparticle was determined based on the maximum zinc content which may not degrade the biocompatibility of the resulting suspension. Particularly, it was determined based on the range of zinc content which may not degrade the biocompatibility as determined by animal tests using sparingly soluble zinc-containing calcium phosphate according to the present invention. We confirmed that up to 12.07% by weight of zinc would not substantially degrade the biocompatibility (Example 7). Based on the findings, the maximum zinc content is set to about 13% by weight since it may be contemplated that the difference between the results obtained with 12.07% and 13% by weight of zinc might be subtle. This range of zinc content may ensure safeness.

Further, the sparingly soluble zinc-containing calcium phosphate with zinc content of the above-described range may be used in combination with any osteogenic component or components. Examples of such osteogenic component includes compounds such as calcium phosphate or tricalcium phosphate as well as calcium formulations such as calcium carbonate, calcium lactate or calcium gluconate. Osteogenic components further include osteogenic vitamin-D, $\alpha$ -calcidol, estrogen-related preparations, calcitonin, bisphosphonate and the like.

A precipitate of previously prepared sparingly soluble zinc-containing calcium phosphate compound with zinc content of the above-described range, dry product thereof, optionally sintered product thereof, and/or microparticle thereof may be mixed with any dry calcium compound or compounds (such as calcium phosphate, tricalcium phosphate, calcium carbonate, calcium lactate, calcium gluconate or the like), optionally sintered product thereof and/or microparticle thereof, in order to produce a sparingly soluble zinc-containing calcium phosphate compound which contains microparticle additive of the calcium compound or compounds at a predetermined range of content.

The thus obtained microparticle of sparingly soluble zinc-containing calcium phosphate may be dispersed and suspended in a liquid media to prepare a suspension or a particle-solvent mixture system. Further, osteogenic vitamin -D, $\alpha$-calcidol, estrogen-related preparations, calcitonin and/or bisphosphonate may be added to the liquid media to obtain a suspension or a particle-solvent mixture system which contains the predetermined amounts of these additives.

Sparingly soluble zinc-containing calcium phosphate can be then administered to a human or animal after dispersing the microparticles of the compound in a liquid media. Thus, it is important for the present invention to disperse and suspend the microparticles of the compound in a liquid for more than a few seconds.

In this way, sparingly soluble zinc-containing calcium phosphate may be kept dispersed in solvent at least during medical treatment such as injection.

This may enable direct administration *in vivo* of a suspension or particle-solvent mixture system in a dosage form such as formulations for oral or parenteral administration, plasters, ointments, suppositories or the like. Such formulations can have high biocompatibility because of slow release of zinc from the microparticles.

The amount of the microparticle of sparingly soluble zinc-containing calcium phosphate to be dispersed and suspended in a liquid media may be calculated from the amount of zinc required and the zinc content of the sparingly soluble zinc-containing calcium phosphate. The calculated amount is a dose of microparticles.

Based on the calculated dose, the liquid media may be suitably determined such that the sedimentation velocity of the microparticle, which can be determined by taking into consideration the particle size of the microparticle and the viscosity of the liquid used as described below, may fall within a target range.

The microparticle and liquid media may be mixed at such a ratio that the resulting suspension or particle-solvent mixture system containing sparingly soluble zinc-containing calcium phosphate may have a suitable viscosity so that it can be used for treatment by, for example, administration thereof.

In other words, the total viscosity of the suspension or particle-solvent mixture system may be selected such that it is kept at 3000Pa s or lower, and preferably 300Pa s or lower, for 2 seconds or longer, and preferably for 10 seconds or longer at room temperature. Lower viscosity may also be used. Suspension or particle-solvent mixture system having almost the same viscosity as that of water can also be used with no or little problem. Therefore, the lower limit of the viscosity may be set to 1.00mPa s, which corresponds to that of water at 20°C.

The upper limit of viscosity was set to 3000Pa s by taking into consideration the following factors: the viscosity of 3000Pa s corresponds to the melt viscosity of MXD6-G (a polyamide having a molecular weight of 40,000) at 260°C; a fluid having a higher viscosity than 3000Pa s may not have enough fluidity to flow through the bore of a syringe needle or catheter even when pressurized nor, unlike an ointment, be deformed by pressing with hands. As apparent from above, a suspension or particle-solvent mixture system having a viscosity over 3000Pa s cannot be injected to a body by using a syringe or catheter, nor be used as a plaster or ointment.

It should be noted that, in the thus-obtained suspension or particle-solvent mixture system according to the present invention which comprises sparingly soluble zinc-containing calcium phosphate dispersed and suspended in a solvent, the sparingly soluble zinc-containing calcium phosphate should be kept suspended in the solvent for 2 seconds or longer, and preferably for 10 seconds or longer. When administered to an animal or human, it is required that the microparticles should be dispersed without sedimentation. Therefore, sparingly soluble zinc-containing calcium phosphate should be kept dispersed in the solvent for a predetermined period of time when administered, whereby it can be administered to an animal or human.

For example, a composition comprising sparingly soluble zinc-containing calcium phosphate dispersed and suspended in a solvent can be poured or filled in a container for oral dosage (a cup) or a syringe for injection. Such a container or syringe may have a length or depth of up to about 8cm. The composition of sparingly soluble zinc-containing calcium phosphate may be prepared so that it has a particle sedimentation velocity (particle herein refers to microparticle of sparingly soluble zinc-containing calcium phosphate) of 4cm/s or less, and preferably 0.8cm/s or less. The sedimentation velocity depends on specific gravity and particle size of the sparingly soluble zinc-containing calcium phosphate, as well as viscosity of the liquid media. Therefore, the sedimentation velocity can be controlled through these factors. In this way, it may be kept suspended in the solvent in the container for 2 seconds or longer, and preferably 10 seconds or longer. This may ensure the time required for administration to a human or animal.

Liquid media for solvent may be selected which can provide a viscosity of 3000Pa s or less, and preferably 300Pa s or less of the resulting suspension or particle-solvent mixture system in order to ensure the fluidity of the resulting suspension or particle-solvent mixture system as described above. Particular examples of such liquid may include: water-miscible solvents such as physiological saline, aqueous solution of 2.5% by weight or less of sodium chloride, Ringer's solution, purified water, distilled water for injection, distilled water, physiological salt solution, propylene glycol and ethanol; water-immiscible solvents such as triglyceride, safflower oil, soybean oil, sesame oil, rape seed oil and peanut oil; and polyethylene glycols (Macrogol). Propylene glycol or ethanol may be mixed with an appropriate amount of physiological saline, Ringer's solution, purified water, distilled water for injection or distilled water prior to use.

In this way, a suspension or particle solvent mixture system comprising sparingly soluble zinc-containing calcium phosphate for sustained release of zinc can be obtained which can be administered orally or parenterally without requiring any surgical operation, have high biocompatibility and provide long-lasting sustained release of zinc.

Particle sedimentation velocity (particle herein refers to microparticle of sparingly soluble zinc-containing calcium phosphate) can be measured under these conditions.

An equation can be derived by using Stokes' law as described below. Particle sedimentation velocity can be calculated by using this equation. Briefly, the particle size and the specific gravity and viscosity of the solvent may be used to calculate the particle sedimentation velocity. Particle sedimentation velocity (V) can be calculated from the radius of particle (r), the specific gravity of solvent (d1), the specific gravity of particle (d2), the viscosity of solvent (n) and the gravitational acceleration (g) as follows:

$$V = 2gr^2 (d2 - d1)/9n.$$

Conversely, a solvent can be selected using the above-described equation by substituting any desirable parameter (V) within the above-described range (particle sedimentation velocity of 4cm/s or less, preferably 0.8cm/s or less), an experimentally determined particle size (e.g., an average particle size determined from particle-size distribution) and specific gravity of the particle. In other words, a linear relationship can be obtained between the specific gravity and viscosity of solvent as described below by using the above-described equation and substituting values of sedimentation velocity (V), particle radius (r) and specific gravity of a particle (d1) as follows:

$$d1 = d2 - (9V/2gr^2)n = A \cdot B \cdot n$$

wherein A and B are constants. Selecting a solvent having a specific gravity (d1) and viscosity (n) conforming to the above-described equation may lead to obtain target sedimentation velocity.

In this way, a combination of particle size and solvent can be determined such that the resulting suspension has a particle sedimentation velocity of 4cm/s or less (preferably 0.8cm/s or less).

Although these equations have been derived on the premise that Stokes' law can be applied to a liquid which shows Newtonian flow, these may also be applicable to a liquid which shows non-Newtonian flow without causing any substantial problems since the range of sedimentation velocity is so small.

When a transparent solvent is used, a suspension may be irradiated with a semiconductor laser beam to determine sedimentation velocity from the time required until the optical path becomes visible. For irradiation, a semiconductor laser of less than 1mW (JIS class II 650nm) may be used. First, the minimum concentration of a particle in suspension may be determined at which the incident laser beam is completely scattered so that no optical path is visible. Next, the suspension at the minimum particle concentration may be left to stand in order to allow some suspended particles to sediment with time. The sedimentation of particles will allow the incident laser beam to transmit through the suspension so that a linear optical path will appear (Tyndall effect).

In summary, the sedimentation velocity of a particle can be calculated from the time of particle sedimentation determined by irradiating the suspension with a semiconductor laser of less than 1mW (JIS class II 650nm) and determining the time required until the optical path of the incident laser transmitting through the suspension become visible, and particle sedimentation distance.

Effects of sparingly soluble zinc-containing calcium phosphate according to the present invention can be confirmed as follows.

Effects of sustained release of zinc can be assayed by comparing the result obtained by administration of sparingly soluble zinc-containing calcium phosphate according to the present invention with one obtained by administration of zinc-free calcium phosphate.

Further, biocompatibility may also be confirmed by observing the cells or tissues to which sparingly soluble zinc-containing calcium phosphate has been administered.

Results obtained by administration of sparingly soluble zinc-containing calcium phosphate may be compared with those obtained by administration of zinc-free calcium phosphate as described below.

A comparison may be performed among groups of rats which received a normal diet (group N); those that received injection of a calcium phosphate with different amounts of zinc (groups D1 and D2); and those that received injection of zinc-free β-tricalcium phosphate (group D3).

Particularly, female Wistar rats (for example, 5 weeks) are divided to prepare 4 groups and bred, grown (for example, for 9 weeks) using the following conditions to induce disease:

> group N: without ovariectomy, received normal diet;
> group D1 : ovariectomy, received diet with low levels of zinc and vitamin D;
> group D2 : ovariectomy, received diet with low levels of zinc and vitamin D; and
> group D3 : ovariectomy, received diet with low levels of zinc and vitamin D.

By the 9th week, rats of groups D1-D3 will develop zinc-deficiency-type osteoporosis. Next, rats in groups N and D1 "receive a diet supplemented with Ca and an intramuscular injection of a suspension of microparticles of zinc-containing β-tricalcium phosphate containing 6.17% by weight of zinc", group D2 "receives a diet supplemented with Ca and an intramuscular injection of suspension of microparticles of zinc-containing β-tricalcium phosphate containing 12.05% by weight of zinc" and group D3 "receives a diet supplemented with Ca and an intramuscular injection of a suspension of zinc-free β-tricalcium phosphate". Supplying a diet supplemented with Ca corresponds to administration of osteogenic materials, e.g., oral administration of a calcium containing preparation to a human.

Group N will be kept in a normal condition while group D3 will be still in condition of zinc-deficiency-type osteoporosis. The ability of a suspension of sparingly soluble zinc-containing calcium phosphate (groups D1 and D2) to make the rats recover from zinc-deficiency-type osteoporosis can be evaluated by comparing conditions of rats between groups D1-D3 and N.

A suspension may be administered intramuscularly at the left thigh once a week for each group. Each suspension contains 10mg of microparticles having a particle size of 38 microns or smaller suspended in 0.2ml of physiological saline solution.

At 18 weeks (i.e., 9 weeks after intramuscular injection started), bone density is compared between group D3, which has been injected with a suspension of zinc-free β-tricalcium phosphate, and groups D1 and D2, which have been administered with a suspension of zinc-containing β -tricalcium phosphate.

In this way, the ability of sparingly soluble zinc-containing calcium phosphate to cure animal or patient of zinc-deficiency-type osteoporosis locally through zinc slowly released from the sparingly soluble zinc-containing calcium phosphate and possibly to prevent bone fracture may be examined by administering a suspension of sparingly soluble zinc-containing calcium phosphate in the vicinity of the site affected by zinc-deficiency-type osteoporosis.

Both right and left thigh bones were removed from the rats at 18 weeks (i.e., 9 weeks after intramuscular injection started), and determined for three-point bending strength of the bones by a three-point bending test using an Instron type universal tester.

Particularly, failure strength is determined by a three-point bending test in the thigh bones from the rats of group N and rats of group D3, to which a suspension containing zinc-free β-tricalcium phosphate has been injected. On the other hand, failure strengths of thigh bones from rats in group D1, which has been injected with a suspension of microparticles of zinc-containing β -tricalcium phosphate containing 6.17% by weight of zinc, and from rats in group D2, which has been injected with a suspension of microparticles of zinc-containing β-tricalcium phosphate containing 12.05% by weight of zinc, are determined. In this way, the effect on improvement in bone strength through administering a suspension of sparingly soluble zinc-containing calcium phosphate in the vicinity of the bones affected by zinc-deficiency-type osteoporosis can be examined.

Next, it may be examined if there is any flush or alopecia in and around the site where sparingly soluble zinc-containing calcium phosphate has been administered. Further, the muscular tissue in the vicinity of the affected bones may be dissected to examine if there is any inflammatory exudate, inflammation or granulation tissue, thereby evaluating the biocompatibility of the sparingly soluble zinc-containing calcium phosphate.

A suspension or particle-solvent mixture system which contains microparticles of the sparingly soluble zinc-containing calcium phosphate compound according to the present invention is characterized in that the sparingly soluble zinc-containing calcium phosphate has high biocompatibility and can compensate for a shortage of zinc efficiently over a long period of time and in that the microparticles are suspended or dispersed in a water-miscible or immiscible solvent. These characteristics enable oral or parenteral local administration of the suspension or particle-solvent mixture. The suspension or particle-solvent mixture system can act as a drug which has high biocompatibility with tissues with which the drug is brought into contact. A suspension or particle-solvent mixture system containing a higher amount of zinc may have greater effects on zinc deficiency.

A suspension or particle-solvent mixture system containing microparticles of a smaller size which consist of zinc-containing calcium phosphate compound, may release zinc faster, while one containing microparticles of greater size may release zinc slowly.

Further, a sparingly soluble zinc-containing calcium phosphate compound may be used in combination with any calcium containing preparation to enhance the ability of the drug to treat osteoporosis.

Additionally, local treatment may be possible by administering sparingly soluble zinc-containing calcium phosphate compound in the vicinity of a bone, for example, by injection.

Hereinafter, the present invention will be described in more detail in reference to the following examples though they are not intended to limit the scope of the invention.

Examples

Example 1

Ca(OH)$_2$ (3.24mol), H$_3$PO$_4$ (2.40 mol) and Zn(NO$_3$)$_2$•6H$_2$O (0.360mol) were added to extra pure water, and the mixture was stirred to obtain a precipitate.

The precipitate was collected by filtration and dried to obtain a dry product which was then heated at 850°C and granulated to give calcium phosphate powder which contained 12.07% by weight of zinc.

It was confirmed by X-ray diffraction that the zinc-containing calcium phosphate powder was a mixture of zinc-containing β -tricalcium phosphate and CaZn$_2$(PO$_4$)$_2$.

The zinc-containing calcium phosphate powder was mixed with pure tricalcium phosphate powder, heated at 850°C for 5 hours followed by heating at 1000°C for 5 hours to give a powdery product which contained from 316 ppm to 6.17% by weight of zinc. It was confirmed that the zinc-containing calcium phosphate powder was zinc-containing β-tricalcium phosphate.

The thus obtained powder was classified and suspended in pseudo body fluid, physiological saline solution, distilled water, rape seed oil or alcohol to the ratio of 2mg solid/ml liquid, and the sedimentation velocity of particles was determined.

The suspension or particle-solvent mixture system having the above-described solid-liquid ratio was irradiated with a semiconductor laser of less than 1 mW (JIS class II 650nm) at an optical path length of 10mm. During all particles were suspended, no optical path was visible in the suspension or particle-solvent mixture system, since the incident laser was completely diffused. After part of suspending particles sedimented with time, the suspension or particle-solvent mixture system was irradiated with a semiconductor laser of less than 1 mW (JIS class II 650nm) and then the incident laser was able to transmit through the suspension or particle-solvent mixture system and a linear optical line appeared due to the Tyndall effect. The time required until the linear optical line became visible could be measured, and particle sedimentation velocity was calculated from the time measured and the distance of particle sedimentation.

The results are shown in Table 1 below.

Aqueous and alcohol suspensions and particle-solvent mixture systems had a viscosity within the range of 1.0-1.2mPa s, and rape seed oil suspension and particle-solvent mixture system had a viscosity of 200-1000 mPa s, all of which were less than 300Pa s.

Table 1

| Zn content | particle size (μm) | powder wt (mg) | Solvent | Solvent vol. (ml) | Sedimentation velocity (cm/s) |
|---|---|---|---|---|---|
| 6.17% | 60-35 | 10 | pseudo body fluid | 0.5 | 0.65 |
| 6.17% | 35 or smaller | 10 | pseudo body fluid | 0.5 | 0.33 |
| 12.05% | 60-35 | 10 | saline | 0.5 | 0.55 |
| 6.17% | 60-35 | 10 | saline | 0.5 | 0.45 |
| 316ppm | 75-35 | 10 | distilled water | 0.5 | 0.05 |
| 316ppm | 35 or smaller | 10 | distilled water | 0.5 | 0.02 |
| 316ppm | 75 or larger | 10 | rape seed oil | 0.5 | 0.12 |
| 316ppm | 75-35 | 10 | rape seed oil | 0.5 | 0.01 |
| 316ppm | 35 or smaller | 10 | rape seed oil | 0.5 | 0.006 |
| 316ppm | 75-35 | 10 | alcohol | 0.5 | 0.023 |
| 316ppm | 35 or smaller | 10 | alcohol | 0.5 | 0.015 |

Table 1 above shows the behaviors of a variety of suspensions or particle-solvent mixture systems, which contained microparticles of zinc-containing β-tricalcium phosphate dispersed and suspended in different solvents. Sedimentation velocity was 0.8cm/s or lower for all of these suspensions, indicating that these suspensions can be administered orally or parenterally.

Example 2

$Ca(OH)_2$, $H_3PO_4$ and $Zn(NO_3)_2 \cdot 6H_2O$ were added to extra pure water such that zinc was present at an amount of from 129 ppm to 0.129% by weight and the atomic ratio of (Ca+Zn)/P was 1.67 in the resulting product. The mixture was stirred to obtain precipitate which was then collected by filtration, dried and heated at 850°C.

Resultant products were granulated to give zinc-containing hydroxyapatite powders each containing from 129 ppm to 0.129% by weight of zinc.

The zinc-containing hydroxyapatite powder was sieved with 200 mesh (pore size: 75 μm) and suspended, alone or in combination with pure hydroxyapatite and tricalcium phosphate microparticle, in distilled water, rape seed oil, 35%PEG solution or alcohol at a ratio of 2mg solid/ml liquid, and suspending time was determined. The suspending time was determined by irradiating the suspension with a semiconductor laser of less than 1mW (JIS class II 650nm) and determining the time required until the incident laser transmitting through the suspension could be observed by eye. The results are shown in Table 2 below. Viscosity was as follows: 1.0-1.2mPa s for distilled water and alcohol; 200-1000 mPa s for rape seed oil; and 150-300 mPa s for 35 % polyethylene glycol (PEG) solution, all of which were less than 300 Pa s.

Table 2

| Zn content | Particle size (μm) | Powder wt. (mg) | Solvent | Solvent vol.(ml) | Sediment. velocity (cm/s) | Microparticle |
|---|---|---|---|---|---|---|
| 0.129% | 75 or smaller | 10 | distilled water | 0.5 | 0.03 | ZnHAP |
| 0.129% | 75 or smaller | 10 | rape seed oil | 0.5 | 0.015 | ZnHAP |
| 0.129% | 75 or smaller | 10 | 35%PEG | 0.5 | 0.011 | ZnHAP |
| 0.129% | 75 or smaller | 10 | alcohol | 0.5 | 0.25 | ZnHAP |
| 650ppm | 75 or smaller | 10 | distilled water | 0.5 | 0.04 | ZnHAP + HAP |
| 650ppm | 75 or smaller | 10 | rape seed oil | 0.5 | 0.02 | ZnHAP + HAP |
| 650ppm | 75 or smaller | 10 | 35%PEG | 0.5 | 0.013 | ZnHAP + HAP |
| 650ppm | 75 or smaller | 10 | alcohol | 0.5 | 0.18 | ZnHAP + HAP |
| 129ppm | 75 or smaller | 10 | distilled water | 0.5 | 0.04 | ZnHAP |
| 129ppm | 75 or smaller | 10 | rape seed oil | 0.5 | 0.035 | ZnHAP |
| 129ppm | 75 or smaller | 10 | 35%PEG | 0.5 | 0.008 | ZnHAP |
| 129ppm | 75 or smaller | 10 | alcohol | 0.5 | 0.166 | ZnHAP |
| 129ppm | 75 or smaller | 10 | distilled water | 0.5 | 0.045 | ZnHAP + TCP |
| 129ppm | 75 or smaller | 10 | rape seed oil | 0.5 | 0.04 | ZnHAP + TCP |
| 129ppm | 75 or smaller | 10 | 35%PEG | 0.5 | 0.01 | ZnHAP + TCP |
| 129ppm | 75 or smaller | 10 | alcohol | 0.5 | 0.12 | ZnHAP + TCP |

ZnHAP : zinc-containing hydroxyapatite;
HAP : zinc-free hydroxyapatite;
TCP : zinc-free tricalcium phosphate; and
PEG : polyethylene glycol.

Table 2 above shows the behaviors of a variety of suspensions and particle-solvent mixture systems, which contained microparticles of zinc-containing hydroxyapatite, zinc-containing hydroxyapatite and tricalcium phosphate, or zinc-containing hydroxyapatite and hydroxyapatite dispersed and suspended in different solvents. Sedimentation velocity was 0.8cm/s or lower for all of these suspensions and particle-solvent mixture systems, indicating that these suspensions and particle-solvent mixture systems can be administered orally or parenterally.

Example 3

$Ca(OH)_2$, $H_3PO_4$ and $Zn(NO_3)_2 \cdot 6H_2O$ were added to extra pure water so that zinc was present at an amount of 65 ppm by weight and the atomic ratio of (Ca+Zn)/P was 1.67 in the resulting product to obtain precipitate.
The precipitate was collected by filtration, dried and granulated to obtain 65ppm of zinc-containing poorly-crystallized hydroxyapatite powder, which was then classified and suspended in distilled water, rape seed oil or 35%PEG solution at a ratio of 2mg solid/ml liquid, and suspending time was determined as described above.
The suspending time was determined by irradiating the suspension or particle-solvent mixture system with a semiconductor laser of less than 1mW (JIS class II 650nm) and determining the time required until the optical path of the incident semiconductor laser transmitting through the suspension or particle-solvent mixture system could be observed by eye. The results are shown in Table 3 below. Viscosity was as follows: 1.0-1.2mPa s for distilled water; 200-1000 mPa s for rape seed oil; and 150-300 mPa s for 35% PEG solution, all of which were less than 300Pa s.

Table 3

| Zn content | Particle size (μm) | Powder wt. (mg) | Solvent | Solvent vol.(ml) | Sedimentation velocity (cm/s) |
|---|---|---|---|---|---|
| 65ppm | 75 or larger | 10 | distilled water | 0.5 | 0.25 |
| 65ppm | 75-38 | 10 | distilled water | 0.5 | 0.041 |
| 65ppm | 38 or smaller | 10 | distilled water | 0.5 | 0.041 |
| 65ppm | 75 or larger | 10 | rape seed oil | 0.5 | 0.25 |
| 65ppm | 75-38 | 10 | rape seed oil | 0.5 | 0.05 |
| 65ppm | 38 or smaller | 10 | rape seed oil | 0.5 | 0.021 |

Table 3   (continued)

| Zn content | Particle size (μm) | Powder wt. (mg) | Solvent | Solvent vol.(ml) | Sedimentation velocity (cm/s) |
|---|---|---|---|---|---|
| 65ppm | 75 or larger | 10 | 35%PEG | 0.5 | 0.25 |
| 65ppm | 75-38 | 10 | 35%PEG | 0.5 | 0.014 |
| 65ppm | 38 or smaller | 10 | 35%PEG | 0.5 | 0.013 |
| PEG : polyethylene glycol. | | | | | |

Table 3 above shows the behaviors of a variety of suspensions and particle-solvent mixture systems, which contained microparticles of zinc-containing poorly-crystallized hydroxyapatite dispersed and suspended in distilled water, rape seed oil or polyethylene glycol solution. Sedimentation velocity was 0.8cm/s or lower for all of these suspensions, indicating that these suspensions can be administered orally or parenterally.

Example 4

$Ca(OH)_2$, $H_3PO_4$ and $Zn(NO_3)_2$ were mixed and added to extra pure water so that the atomic ratio of (Ca+Zn)/P was 1.50 in the resulting product, and the mixture was stirred to obtain precipitate.

The precipitate was collected by filtration, dried, granulated and heated at 1400°C for 5 hours to obtain zinc-containing α-tricalcium phosphate, which was then classified to collect those having a particle size of 38-106 microns and suspended in distilled water or rape seed oil at a ratio of 2mg solid/ml liquid, and suspending time was determined. The suspending time was determined by irradiating the particle-solvent mixture system with a semiconductor laser of less than 1mW (JIS class II 650nm) and determining the time required until the optical path of the incident laser transmitting through the particle-solvent mixture system could be observed by eye. The results are shown in Table 4 below. Viscosity was as follows: 1.0-1.2mPa s for distilled water; and 200-1000mPa s for rape seed oil, both of which were less than 300 Pa s.

Table 4

| Zn content | Particle size (μm) | Powder wt. (mg) | Solvent | Solvent vol. (ml) | Sedimentation velocity (cm/s) |
|---|---|---|---|---|---|
| 0.126% | 38-106 | 10 | distilled water | 0.5 | 0.25 |
| 0.126% | 38-106 | 10 | distilled water | 0.5 | 0.166 |
| 0.568% | 38-106 | 10 | distilled water | 0.5 | 0.166 |
| 0.568% | 38-106 | 10 | rape seed oil | 0.5 | 0.25 |

Table 4 above shows the behaviors of particle-solvent mixture systems, which contained microparticles of zinc-containing α-tricalcium phosphate dispersed and suspended in distilled water or rape seed oil. Sedimentation velocity was 0.8cm/s or lower for all of these suspensions, indicating that these particle-solvent mixture systems can be administered orally or parenterally.

Example 5

$H_3PO_4$ (4.89g, equivalent to 5.76g of 85% phosphoric acid) was added to water (87.75g) to obtain a diluted phosphoric acid solution. Then, calcium carbonate (5g) and zinc nitrate hexahydrate (0.06-4.095g) were added to the solution at room temperature. The molar ratio of phosphoric acid to calcium in the solution was 1:1. After the mixture was continuously stirred for 12 hours, precipitate was collected by filtration and dried at room temperature. It was confirmed by X-ray diffraction for powder that zinc-containing calcium hydrogenphosphate dihydrate was obtained when 0.25g or less of zinc nitrate hexahydrate was added, while zinc-containing calcium hydrogenphosphate dihydrate and $CaZn_2(PO_4)_2 \cdot nH_2O(n=2)$ were obtained when more than 0.25g of zinc nitrate hexahydrate was added. These powdery products were classified to collect those having a particle size of 38 microns or smaller, and optionally mixed with 50% by weight of calcium carbonate powder, and suspended in rape seed oil, 35% polyethylene glycol (PEG) solution or 60% polyethylene glycol (PEG) solution at a ratio of 2mg solid/ml liquid, and suspending time was determined. The suspending time was determined by irradiating the suspension with a semiconductor laser of less than 1 mW (JIS class II 650nm) and determining the time required until the optical path of the incident laser transmitting through the suspension could be observed by eye. The results are shown in Table 5 below. Viscosity was as follows: 200-1000mPa s for rape seed oil; 150-300mPa s for 35% PEG; and 1-3Pa s for 60% PEG, all of which were less than 300 Pa s.

Table 5

| Zn content | Particle size (µm) | Powder wt. (mg) | Solvent | Solvent vol. (ml) | Sediment. velocity (cm/s) | Microparticle |
|---|---|---|---|---|---|---|
| 0.15 % | 38 or smaller | 10 | rape seed oil | 0.5 | <0.01 | ZnDCPD |
| 0.15 % | 38 or smaller | 10 | 35%PEG | 0.5 | <0.01 | ZnDCPD |
| 0.15 % | 38 or smaller | 10 | 60%PEG | 0.5 | <0.01 | ZnDCPD |
| 1.10 % | 38 or smaller | 15 | rape seed oil | 0.75 | <0.01 | ZnDCPD+SH+CC |
| 1.10 % | 38 or smaller | 15 | 35%PEG | 0.75 | <0.01 | ZnDCPD+SH+CC |
| 1.10 % | 38 or smaller | 15 | 60%PEG | 0.75 | <0.01 | ZnDCPD+SH+CC |
| 12.0 % | 38 or smaller | 15 | rape seed oil | 0.75 | <0.01 | ZnDCPD+SH+CC |
| 12.0 % | 38 or smaller | 15 | 35%PEG | 0.75 | <0.01 | ZnDCPD+SH+CC |
| 12.0 % | 38 or smaller | 15 | 60%PEG | 0.75 | <0.01 | ZnDCPD+SH+CC |

ZnDCPD : zinc-containing calcium hydrogenphosphate;
SH : $CaZn_2(PO_4)_2 \cdot nH_2O(n=2)$; and
CC: calcium carbonate.

Table 5 above shows the behaviors of a variety of suspensions, which contained microparticles of zinc-containing calcium hydrogenphosphate, zinc-containing calcium hydrogenphosphate and calcium carbonate, or zinc-containing calcium hydrogenphosphate and $CaZn_2(PO_4)_2 \cdot nH_2O(n=2)$ and calcium carbonate, dispersed and suspended in rape seed oil, 35% PEG or 60% PEG. Sedimentation velocity was 0.8cm/s or lower for all of these suspensions, indicating that these suspensions can be administered orally or parenterally.

Example 6

Wistar rats (5 weeks, female) were used to prepare 4 groups as follows and breeded for 9 weeks:

group N: without ovariectomy, received normal diet;
group D1 : ovariectomy, received diet with low levels of zinc and vitamin D;
group D2 : ovariectomy, received diet with low levels of zinc and vitamin D; and
group D3 : ovariectomy, received diet with low levels of zinc and vitamin D

The rats of groups D1-D3 developed zinc-deficiency-type osteoporosis by the 9th week. Next, rats in groups N and D1 "received a diet supplemented with Ca and an intramuscular injection of suspension of micropaticles of zinc-containing β -tricalcium phosphate containing 6.17% by weight of zinc".

Likewise, group D2 "received a diet supplemented with Ca and an intramuscular injection of suspension of microparticles of zinc-containing β -tricalcium phosphate containing 12.05% by weight of zinc".

Likewise, group D3 "received a diet supplemented with Ca and an intramuscular injection of a suspension containing zinc-free β-tricalcium phosphate". Supplying a diet supplemented with Ca corresponds to oral administration of a calcium containing preparation.

As a result, group N was kept in a normal condition while group D3 was still in condition of zinc-deficiency-type osteoporosis.

The ability of suspensions of sparingly soluble zinc-containing calcium phosphate (groups D1 and D2) to cure animals of zinc-deficiency-type osteoporosis was evaluated by comparing conditions of rats between groups D1-D3 and N.

Suspensions were injected intramuscularly at the left thigh once a week for each group. Each suspension contained 10mg of micropaiticles having a particle size of 38 microns or smaller suspended in 0.2ml of physiological saline solution.

Results were as follows.

At 18 weeks (i.e., 9 weeks after intramuscular injection started), the bone mineral density of the left-thighs from rats of group D3, which had been injected with zinc-free β -tricalcium phosphate suspension, was 73% relative to that of group N (100%).

On the other hand, the bone mineral density (left-thigh) of rats in group D1, which had been injected with a suspension containing zinc-containing β -tricalcium phosphate containing 6.17% by weight of zinc, was improved to 84% relative to group N (100%), which was statistically significant (p<0.05).

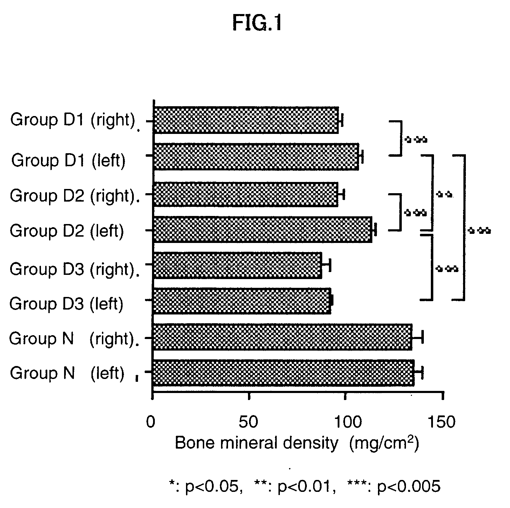

Moreover, the bone mineral density (left-thigh) of rats in group D2, which had been injected with a suspension containing zinc-containing calcium phosphate containing 12.05% by weight of zinc, was further improved to 90% relative to group N (100%), which was also statistically significant (p<0.005) (Figure 1).

Bone mineral density of the right thigh, to which a suspension was not injected, was significantly lower than that of the left thigh.

These results indicate that:

- administration of Ca alone will not help to cure animal or patient of zinc-deficiency-type osteoporosis; and
- zinc-deficiency-type osteoporosis can be locally ameliorated by administration of Ca followed by local administration of a suspension of sparingly soluble zinc-containing calcium phosphate in the vicinity of the bone affected by zinc-deficiency-type osteoporosis, which provides sustained release of zinc from the sparingly soluble zinc-containing calcium phosphate. These results suggest the possibility that such a suspension is able to prevent bone fracture.

Example 7

Thigh bones were removed from the rats in each group prepared in Example 6 at 18 weeks (i.e., 9 weeks after intramuscular injection started), and determined for three-point bending strength by a three-point bending test using an Instron type universal tester. As a result, rats in group D3, to which a suspension containing zinc-free β-tricalcium phosphate had been injected, had a failure strength of 64% relative to that of groups N (100%).

On the other hand, failure strength of left-thigh bone from rats in group D1, to which a suspension of zinc-containing β-tricalcium phosphate containing 6.17% by weight of zinc had been injected, was improved to 76% relative to group N (100%), which was statistically significant (p<0.05).

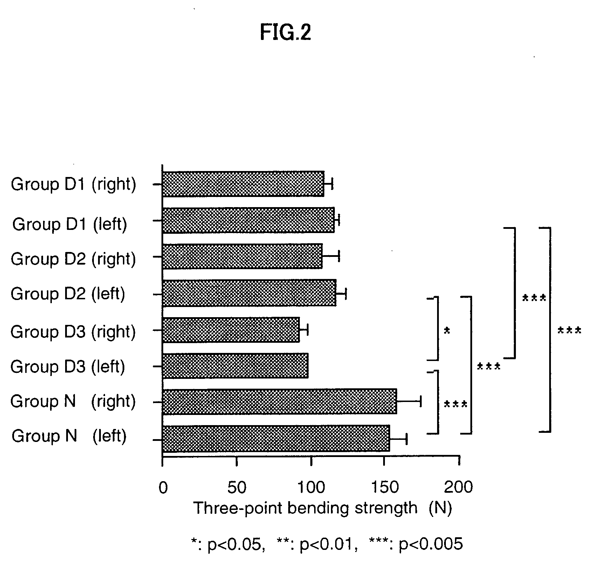

Failure strength of left-thigh bone from rats in group D2, to which a suspension of zinc-containing calcium phosphate containing 12.05% by weight of zinc had been injected, was also improved up to 76% relative to group N (100%), which was statistically significant (p<0.005) (Figure 2).

These results indicate that:

- administration of Ca alone will not substantially help to cure animal or patient of zinc-deficiency-type osteoporosis; and
- zinc-deficiency-type osteoporosis can be effectively ameliorated by administration of Ca followed by local administration of a suspension of sparingly soluble zinc-containing calcium phosphate in the vicinity of the bone affected by zinc-deficiency-type osteoporosis, which provides sustained release of zinc from the sparingly soluble zinc-containing calcium phosphate. These results show the ability of the suspension to improve bone strength.

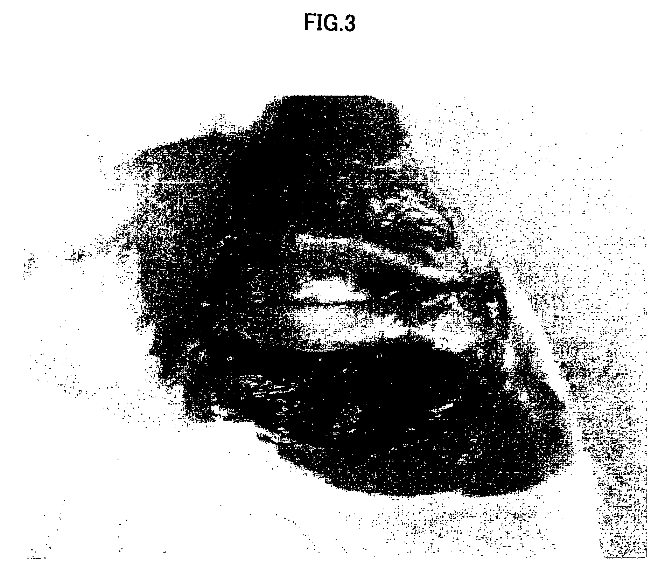

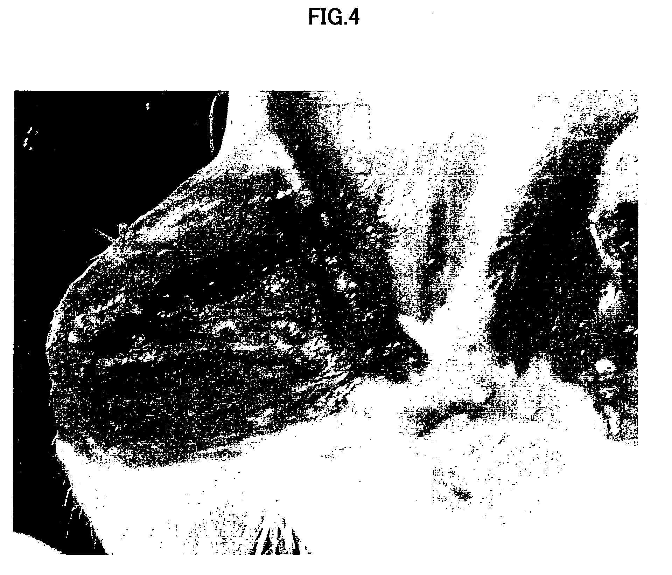

Also, there were no abnormal symptoms such as flush or alopecia on the skin of the site where the suspension was injected. The muscular tissues to which the suspension had been injected were dissected to find no inflammation, inflammatory exudate or granulation tissue in the tissues with which suspension had been brought into contact. No difference was observed on the skin or muscular tissues by visual inspection between group D2 (Figure 3), which received a suspension of zinc-containing β-tricalcium phosphate containing 12.05% by weight of zinc, and group D3 (Figure 4), which received a suspension of β-tricalcium phosphate. These results indicate that the suspension of zinc-containing β-tricalcium phosphate containing 12.05% by weight of zinc has biocompatibility equivalent to that of a suspension of highly biocompatible β-tricalcium phosphate, which is widely used clinically as granular or porous ceramic implant for bone tissue.

Industrial Applicability

As described above, zinc-containing calcium phosphate with particular chemical composition, specifically, sparingly soluble zinc-containing calcium phosphate, which has high biocompatibility and provides long-term sustained release of zinc, can be administered orally as well as parenterally by using a suspension or a particle-solvent mixture system which contains the sparingly soluble zinc-containing calcium phosphate according to the present invention. The present invention can also provide zinc-containing preparations with high biocompatibility with the tissue with which the preparation is brought into contact. Thus, the present invention can be used to treat diseases caused by zinc-deficiency.

All publications, patents and patent applications cited herein are incorporated herein by reference in their entirety.

**Claims**

1. A suspension or particle-solvent mixture system which comprises a liquid containing a microparticle of zinc-containing calcium phosphate consisting of from 0.6 ppm to 13% by weight of zinc, 33-57% by weight of $P_2O_5$, 10-65% by weight of CaO and 0-28% by weight of $H_2O$.

2. A suspension or particle-solvent mixture system which comprises a liquid containing a microparticle of zinc-containing calcium phosphate consisting of from 120 ppm to 13% by weight of zinc, 33-57% by weight of $P_2O_5$, 10-65% by weight of CaO and 0-28% by weight of $H_2O$.

3. The suspension or particle-solvent mixture system according to claim 1 or 2, wherein said microparticle of zinc-containing calcium phosphate is one or more compounds selected from the group consisting of zinc-containing hydroxyapatite, zinc-containing poorly-crystallized apatite, zinc-containing $\alpha$-tricalcium phosphate, zinc-containing $\beta$-tricalcium phosphate, zinc-containing calcium hydrogenphosphate, zinc-containing amorphous calcium phosphate and $CaZn_2(PO_4)_2 \cdot nH_2O$ ($0 \leq n \leq 2$).

4. The suspension or particle-solvent mixture system according to any one of claims 1-3, wherein at least one osteogenic compound selected from the group consisting of vitamin D, $\alpha$-calcidol, estrogen-related preparations, calcitonin, bisphosphonate and calcium containing preparations is further contained.

5. The suspension or particle-solvent mixture system according to any one of claims 1-4, wherein said liquid is a water-miscible solvent selected from the group consisting of physiological saline solution, an aqueous solution of 2.5% by weight or less of sodium chloride, Ringer's solution, purified water, distilled water for injection, distilled water, physiological salt solution, propylene glycol, ethanol, and a mixture of propylene glycol or ethanol with any one or more of these solutions or waters.

6. The suspension or particle-solvent mixture system according to any one of claims 1-4, wherein said liquid is selected from the group consisting of water-immiscible solvents including triglyceride, safflower oil, soybean oil, sesame oil, rape seed oil and peanut oil or from polyethylene glycols (Macrogol).

7. The suspension or particle-solvent mixture system according to any one of claims 1-6, wherein said suspension or particle-solvent mixture system is used for a therapeutic agent for treating zinc deficiency.

8. The suspension or particle-solvent mixture system according to claim 4, wherein said suspension or particle-solvent mixture system is used for a therapeutic agent for treating zinc deficiency and osteogenetic agent.

## FIG.1

*: p<0.05, **: p<0.01, ***: p<0.005

# FIG.2

*: p<0.05,  **: p<0.01,  ***: p<0.005

FIG.3

FIG.4